# EUROPEAN PATENT APPLICATION

(11) **EP 3 943 103 A1**
(43) Date of publication of application: **26.01.2022**
(21) Application number: 20382666.4
(22) Date of filing: 24.07.2020
(51) Int. Cl.: A61K 38/47, A61P 31/04, C07K 14/47, C07K 14/005

(54) **POLYPEPTIDES WITH ANTIBACTERIAL ACTIVITY**

(71) Applicant: Consejo Superior de Investigaciones Científicas (CSIC), 28006 Madrid (ES)
(72) Inventor: GARCÍA GONZÁLEZ, Pedro, 28040 Madrid (ES); VÁZQUEZ FERNÁNDEZ, Roberto, 28040 Madrid (ES); GARCÍA LÓPEZ, Ernesto, 28040 Madrid (ES)
(74) Representative: Pons

(57) **Abstract**

The invention relates to a proteinaceous antimicrobials derivates of the polypeptide Pae87, and more particularly, to Pae87F enzybiotic, P87 and P88 polypeptides with bactericidal activity, particularly active against Gram-negative bacteria, such as P. *aeruginosa, A. baumannii and M. catarrhalis.* The invention relates to these polypeptides for use in the treatment of a disease caused by a bacterium, and preferably the disease is a respiratory tract infection.

SEQUENCE LISTING

<110>  Consejo Superior de Investigaciones Cientificas (CSIC)

<120>  Polypeptides with antibacterial activity

<130>  EP1641.1567

<160>  5

<170>  PatentIn version 3.5

<210>  1
<211>  186
<212>  PRT
<213>  Artificial Sequence

<220>
<223>  Pae87

<400>  1

Met Ala Leu Thr Glu Gln Asp Phe Gln Ser Ala Ala Asp Asp Leu Gly
1               5                   10                  15

Val Asp Val Ala Ser Val Lys Ala Val Thr Lys Val Glu Ser Arg Gly
            20                  25                  30

Ser Gly Phe Leu Leu Ser Gly Val Pro Lys Ile Leu Phe Glu Arg His
        35                  40                  45

Trp Met Phe Lys Leu Leu Lys Arg Lys Leu Gly His Asp Pro Glu Ile
    50                  55                  60

Asn Asp Val Cys Asn Pro Lys Ala Gly Gly Tyr Leu Gly Gly Gln Ala
65                  70                  75                  80

Glu His Glu Arg Leu Asp Lys Ala Val Lys Met Asp Arg Asp Cys Ala
                85                  90                  95

Leu Gln Ser Ala Ser Trp Gly Leu Phe Gln Ile Met Gly Phe His Trp
            100                 105                 110

Glu Ala Leu Gly Tyr Ala Ser Val Gln Ala Phe Val Asn Ala Gln Tyr
        115                 120                 125

Ala Ser Glu Gly Ser Gln Leu Asn Thr Phe Val Arg Phe Ile Lys Ile
    130                 135                 140

Asn Pro Ala Ile His Lys Ala Leu Lys Ser Lys Asn Trp Ala Glu Phe
145                 150                 155                 160

Ala Lys Arg Tyr Asn Gly Pro Asp Tyr Lys Lys Asn Asn Tyr Asp Val

165                 170                 175

Lys Leu Ala Glu Ala Tyr Gln Ser Phe Lys
            180                 185

<210>  2
<211>  29
<212>  PRT
<213>  Artificial Sequence

<220>
<223>  P87

<400>  2

Leu Asn Thr Phe Val Arg Phe Ile Lys Ile Asn Pro Ala Ile His Lys
1               5                   10                  15

Ala Leu Lys Ser Lys Asn Trp Ala Glu Phe Ala Lys Arg
            20                  25

<210>  3
<211>  38
<212>  PRT
<213>  Artificial Sequence

<220>
<223>  calcium-binding F domain

<400>  3

Thr Phe Lys Asn Cys Thr Glu Leu Arg Lys Lys Tyr Pro Asn Gly Val
1               5                   10                  15

Pro Ser Ser His Pro Ala Tyr Gln Ser Lys Met Asp Arg Asp His Asp
            20                  25                  30

Asn Tyr Ala Cys Glu Arg
        35

<210>  4
<211>  29
<212>  PRT
<213>  Artificial Sequence

<220>
<223>  P88

<400>  4

Leu Asn Ala Phe Val Arg Phe Ile Lys Lys Ala Pro Ala Ile Phe Lys
1               5                   10                  15

Ala Leu Lys Ser Lys Asn Trp Ala Gln Phe Ala Lys Arg
            20                  25

<210>  5
<211>  230
<212>  PRT
<213>  Artificial Sequence

<220>
<223>  Pae87F

<400>  5

Met Ala Leu Thr Glu Gln Asp Phe Gln Ser Ala Ala Asp Asp Leu Gly
1               5                   10                  15

Val Asp Val Ala Ser Val Lys Ala Val Thr Lys Val Glu Ser Arg Gly
            20                  25                  30

Ser Gly Phe Leu Leu Ser Gly Val Pro Lys Ile Leu Phe Glu Arg His
        35                  40                  45

Trp Met Phe Lys Leu Leu Lys Arg Lys Leu Gly His Asp Pro Glu Ile
    50                  55                  60

Asn Asp Val Cys Asn Pro Lys Ala Gly Gly Tyr Leu Gly Gly Gln Ala
65                  70                  75                  80

Glu His Glu Arg Leu Asp Lys Ala Val Lys Met Asp Arg Asp Cys Ala
                85                  90                  95

Leu Gln Ser Ala Ser Trp Gly Leu Phe Gln Ile Met Gly Phe His Trp
            100                 105                 110

Glu Ala Leu Gly Tyr Ala Ser Val Gln Ala Phe Val Asn Ala Gln Tyr
        115                 120                 125

Ala Ser Glu Gly Ser Gln Leu Asn Thr Phe Val Arg Phe Ile Lys Ile
    130                 135                 140

Asn Pro Ala Ile His Lys Ala Leu Lys Ser Lys Asn Trp Ala Glu Phe
145                 150                 155                 160

Ala Lys Arg Tyr Asn Gly Pro Asp Tyr Lys Lys Asn Asn Tyr Asp Val
                165                 170                 175

Lys Leu Ala Glu Ala Tyr Gln Ser Phe Lys Gly Ser Gly Ser Gly Ser
            180                 185                 190

Thr Phe Lys Asn Cys Thr Glu Leu Arg Lys Lys Tyr Pro Asn Gly Val
        195                 200                 205

Pro Ser Ser His Pro Ala Tyr Gln Ser Lys Met Asp Arg Asp His Asp
    210                 215                 220

Asn Tyr Ala Cys Glu Arg
225                 230

## Description

The invention relates to proteinaceous antimicrobials derivates of the polypeptide Pae87, and more particularly, to Pae87F enzybiotic, P87 and P88 polypeptides from a *Pseudomonas aeruginosa* phage with bactericidal activity, particularly active against Gram-negative bacteria, such as *P. aeruginosa, Acinetobacter baumannii* and *Moraxella catarrhalis.*

### BACKGROUND ART

Lower respiratory tract infections caused about 3.2 million deaths worldwide in 2015 and they remain the deadliest reported diseases. *Streptococcus pneumoniae* is the leading cause for most of the bacterial pneumonia cases in children, as well as for the community-acquired pneumonia in elderly patients with comorbidities (A. Torres et al 2018, RespirMed, 137:6-13). In addition, hospital-acquired pneumonia and ventilator-associated pneumonia are among the leading nosocomial infections worldwide, with an increasing frequency of multidrug resistant (MDR) Gram-negative bacteria (G-), like *Pseudomonas aeruginosa* and *Acinetobacter baumannii* (J.M. Kidd, et al 2018, Expert Opin Pharmacother, 19:397-408).

Concerning public health issue, antimicrobial resistance (AMR) and associated morbidity and mortality data have been increasing globally. In this regard, has been estimated that AMR could produce about 10 million deaths a year by 2050. In this scenario, the World Health Organization has called for global action on AMR, which has encouraged several actions, one of them the search for alternatives to the currently used antibiotics. In particular, interest has been mainly focused against a group of MDR bacteria known as "the ESKAPE bugs" that are of particular concern: "A" is for *A. baumannii* and "P" for *P. aeruginosa (*W.H. Organization, Prioritization of Pathogens to Guide Discovery, Research and Development of New Antibiotics for Drug-Resistant Bacterial Infections, Including Tuberculosis, in, World Health Organization, 2017*).*

A few decades ago, phage therapy has emerged as a promising alternative to conventional antibiotics, encompassing not only the use of entire virions but also some of their gene products. In this context, since the beginning of twenty-first century, phage lysins have been extensively tested as antibacterial agents. These enzymes break specific bonds of the bacterial peptidoglycan and, when added exogenously, provoke a rapid osmotic lysis and death, displaying potent bactericidal activity. This enzymatic activity of lysins was the basis for their exploration as antibacterial agents, also named "enzybiotics" (Nelson D., et al 2001, Proc Natl Acad Sci U S A, 98:4107-4112).

Lysins are usually classified as glycosidases (glucosaminidases, transglycosylases, and lysozymes or muramidases), if they break any of the bonds of the glycan chain; N-acetylmuramoyl-L-alanine amidases (NAM-amidases), if they break the amide bonds between the glycan strands and peptide chains; or endopeptidases, if they hydrolyze different bonds within peptide chains. Lysins possess several advantages over antibiotics: (a) they rapidly kill bacteria, practically upon contact; (b) they can be specific to the target pathogen, particularly against Gram-positive (G+) bacteria, which allows to preserve the normal microbiota (Cheng M., et al 2017, Sci Rep, 7:10164); (c) development of resistance seems very unlikely (Gilmer D.B., et al 2013, Antimicrob Agents Chemother, 57;2743-2750); (d) lysins are active independently of the bacterial physiological state and are effective against MDR bacteria (Briers Y., et al 2014, Antimicrob Agents Chemother, 58;3774-3784); (f) they can act synergistically with other lysins or antibiotics and reduce the development of resistance while increasing therapeutic efficiency; and (g) lysins are also effective killing colonizing pathogens growing on mucosal surfaces and/or in biofilms.

Lysins encoded by phages infecting G+ bacteria generally display a modular structure, comprising one or more catalytic domains and one or more cell wall binding domains, whereas phages from G- bacteria usually encode globular lysins with a single catalytic domain, with several exceptions (Walmagh M., et al 2012, PLoS One, 7; e36991*).*

In general, lysins have showed lesser bactericidal effect against G- bacteria, due to the presence of the outer membrane (OM). For this reason, most recent efforts have been directed toward the engineering of the enzymes themselves, giving rise to the "artilysin" concept (Briers Y., et al 2014, mBio, 5;e01379-01314.), wherein lysins were fused to cationic, antimicrobial peptides (AMPs), and these fusions were able to exert a permeabilizing activity that allowed them to cross *P. aeruginosa* OM to degrade the peptidoglycan layer both *in vitro* and *in vivo.*

Typically, this intrinsic activity from without relies on non-enzymatic mechanisms, which were first described for the T4 phage lysozyme (During K., et al 1999, FEBS Lett, 449;93-100) and then in several *P. aeruginosa* phage lysins (Rotem S., et al 2006, Peptides, 27;18-26). These lysins harbor AMP-like elements (peptides with an amphipathic secondary structure and a positive net charge) that destabilize the OM.

In some cases, as for T4 lysozyme, these regions account for the bactericidal activity of the enzyme to a higher extent than the enzymatic activity itself. One of the first examples of a lysin with a natural cationic peptide exploited as an enzybiotic was the *Bacillus amyloliquefaciens* phage lysin, Lys1521, which was indeed able to lyse *P*. *aeruginosa* cells (Morita M., et al2001, J Biosci Bioeng, 91;469-473). Other examples of *P. aeruginosa* lysins with intrinsic anti-G- activity include OBPgp279 (Walmagh M., et al 2012, PLoS One, 7; e36991) and LysPA26 (Guo M., et al 2017, Front Microbiol, 8;293).

In general, lysins against G- bacteria appear to be less specific than their G+ counterparts, possibly due to the simpler organization of the former sacculi (M.A. de Pedro, et al 2015, Front Microbiol, 6;449). This broader spectrum allows some lysins to kill several pathogenic genera, like the already mentioned lysin LysPA26, which besides *P. aeruginosa* can also lyse other G- pathogens such as *E. coli, K. pneumoniae* or *A. baumannii,* or Art-175, which also kills *A. baumannii* (Briers Y., et al 2014, Antimicrob Agents Chemother, 58;3774-3784).

PlyF307, from an *Acinetobacter phage,* was capable of killing *A. baumannii* isolates, including MDR strains, both in planktonic and biofilm cultures and represents the first example of an intact lysin with intrinsic anti-G- activity tested in a mammalian (mouse bacteremia) model (Lood R., et al 2015, Antimicrob Agents Chemother, 59;1983-1991). Unsurprisingly, it was later determined that such intrinsic activity from without partly resided in a cationic peptide located in the C-terminal domain of the lysin (Thandar M., et al 2016, Antimicrob Agents Chemother, 60;2671-2679).

However, therapeutically relevant lysins with a high broad-spectrum antimicrobial activity are still necessary.

### SUMMARY OF THE INVENTION

The present invention discloses proteinaceous antimicrobials derivates of the polypeptide Pae87 with high antimicrobial activity, particularly against Gram-negative bacteria.

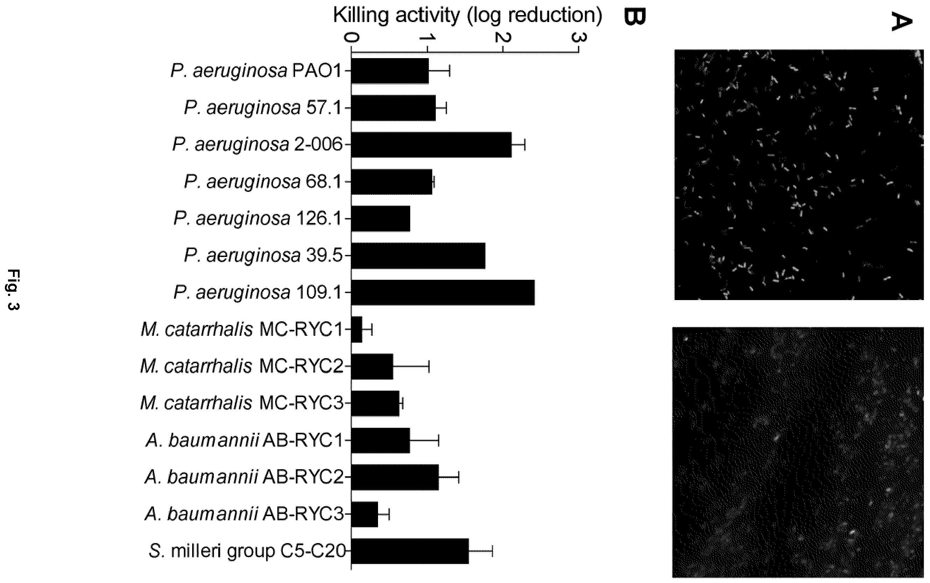

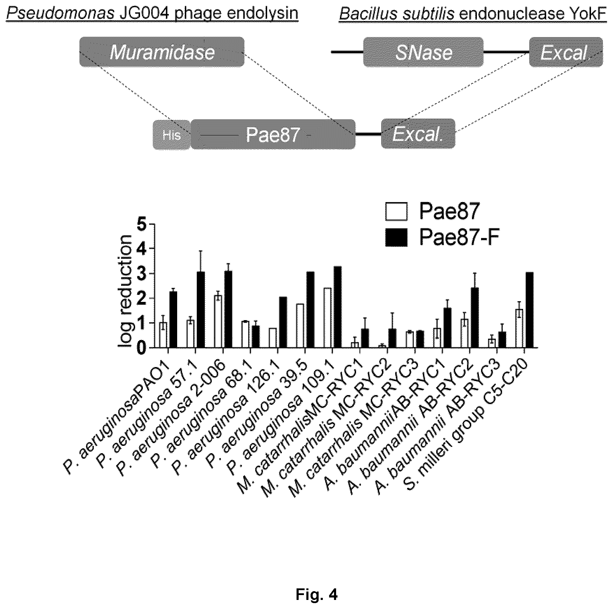

The inventors have analysed lysin sequences with putative antibacterial activity by means of a bioinformatic procedure to screen plausible lysin candidates able to interact with Gram-negative (G-) outer membrane. First, the inventors observed that a lysin from a *Pseudomonas aeruginosa* phage JG004, Pae87 with amino acid sequence SEQ ID NO: 1, have a bactericidal activity, particularly active both against *P. aeruginosa* and *A. baumannii,* as well as other relevant pathogens such as *M*. *catarrhalis* or *Streptococcus milleri* group (SMG) bacteria (Figure 3). Then, they fused Pae87 with a calcium-binding domain derived from *Bacillus subtilis* nuclease YokF (termed 'F', with amino acid sequence SEQ ID NO: 3), resulting molecule Pae87F with amino acid sequence SEQ ID NO: 5, which displayed an improved activity against the same range of bacteria (Figure 4).

Additionally, the inventors defined a putative AMP-like region, based on wild-type Pae87 sequence analysis, and this way obtained antibacterial polypeptide P87 with amino acid sequence SEQ ID NO: 2. Further, the inventors designed and synthesized a novel polypeptide P88, based on P87 polypeptide with five mutations intended to maximize net charge and hydrophobic moment, yielding amino acid sequence SEQ ID NO: 4.

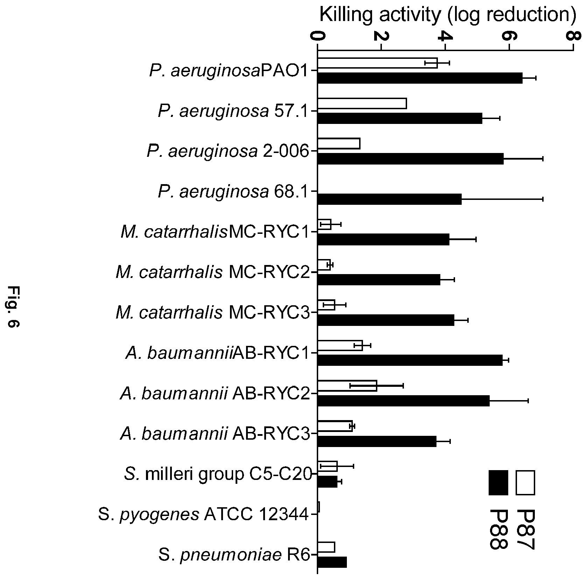

Finally, the inventors tested the bactericidal activity of P87 and P88. P87 had a bactericidal activity, especially against *P. aeruginosa* (up to 4-log killing) and *A. baumannii* (up to 2-log killing), and P88 activity was much higher with a killing activity ranging between 4 and 6 logarithmic units of viable cells reduction in all of the three tested Gram-negative bacteria, *P. aeruginosa, M. catarrhalis and A. baumannii* (Figure 6). So, the inventors have obtained proteinaceous antimicrobials derivates of the polypeptide Pae87; P87, P88 and Pae87F polypeptides, with high antimicrobial activity, particularly against a wider range of Gram-negative bacteria than expected for a *Pseudomonas* lysin.

A first aspect of the present invention related to an isolated polypeptide with antibacterial activity comprising an amino acid sequence with at least 70% identity with the amino acid sequence SEQ ID NO: 2, hereinafter the polypeptide of the invention, wherein the amino acid sequence is not SEQ ID NO: 1.

In the context of the present invention, the term "polypeptide" refers to a polymer with a length of between 8 to 250 amino acids linked via amide bonds that are formed between an amino group of one amino acid and a carboxyl group of another amino acid. The terms "amino acid sequence", "polypeptide", "peptide" and "oligopeptide" are used interchangeably.

The term "isolated" or "purified" means a polypeptide that is removed from at least one component with which it is naturally associated and, consequently, it is substantially free of cellular material or other contaminating polypeptides from the cell or tissue source from which the agent is derived, or substantially free from chemical precursors or other chemicals when chemically synthesized.

The polypeptide of the invention can be obtained by techniques widely known in the state of the art, such as chemical synthesis, genetic recombination, expression of the polynucleotide that encodes the polypeptide of the invention. All of these techniques are routine practice for the skilled in the art.

Additionally, it is common general knowledge that the carboxyl and amino terminal ends of the polypeptide of the invention can be protected against proteolysis. For example, the amino terminal end may be in the form of an acetyl group (Ac) and/or the carboxyl terminal end may be in the form of an amide group (Am). It is also possible to carry out internal modifications of the polypeptides so that they are resistant to proteolysis. Examples of these internal modifications include, without limiting to, modifications in which at least one polypeptide bridge -CONH- is modified and replaced by a reduced bond (CH₂NH), a retroverse bond (NHCO), an oxymethylene bond (CH₂-O), a thiomethylene bond (CH₂-S), a ketomethylene bond (CO-CH₂), a hydroxyethylene bond (CHOH-CH₂), a bond (NN), an E-alkene bond or a -CH=CH- bond. The polypeptides can also be stabilized by intramolecular crossing, for example, by modifying at least two amino acid residues with olefin side chains, preferably C3-C8 alkenyl chains, preferably pentel-2-yl chains, followed by crosslinking chains. All of these polypeptides chemically modified to resist proteolysis are also contemplated within the present invention.

The polypeptide of the invention comprises at least 70% identity with the sequence SEQ ID NO: 2. In the present invention, "identity" or "sequence identity" refers to the degree of similarity between two nucleotide sequences or the amino acids obtained by aligning the two sequences. Depending on the number of common residues between the aligned sequences, a degree of identity is expressed as a percentage. The degree of identity between two parameter sequences can be determined by standard methods, for example, by standard sequence alignment algorithms known in the state of the art, such as BLAST. The BLAST programs, for example, BLASTN, BLASTX and TBLASTX, BLASTP and TBLASTN, are in the public domain on the website of the National Center for Biotechnology Information (NCBI). In a particular embodiment, the polypeptide of the invention comprises an identity of at least 80%, preferably comprises an identity of at least 81, 82, 83, 84, 85, 86, 87, 88, 89, 90, 91, 92, 93, 94, 95, 96, 97, 98 or 99% with the sequence SEQ ID NO: 2. In another more particular embodiment, the polypeptide of the invention comprises a sequence identity of 82% with the sequence SEQ ID NO: 2. In another more particular embodiment, the polypeptide of the invention comprises a sequence identity of 100% with the sequence SEQ ID NO: 2. As understood by the person skilled in the art, all polypeptides that have an identity of at least 70% with the sequence SEQ ID NO: 2, and which have the same function as SEQ ID NO: 2, that is, they have antibacterial activity, they are functionally equivalent variants of the polypeptide of the invention. Assays to identify polypeptides functionally equivalent to the polypeptide of the invention are routine practice for the skilled in the art.

The inventors have tested bactericidal activity of P87 against Gram-negative bacteria. The term "bactericidal activity", "activity against bacteria" or "antimicrobial activity" used interchangeably in the present invention, refers to the capacity or ability to the polypeptide of the invention or polypeptides functionally equivalent to the polypeptide of the invention for preventing, reducing or inhibiting the growth of bacteria or killing bacteria, preferably Gram-negative bacteria.

The bactericidal activity or antibacterial activity of the polypeptide of the invention may be influenced by growth conditions, bacterial density, test duration, and extent of reduction in bacterial numbers. The *in vitro* microbiological techniques to determine the bactericidal activity of the polypeptide of the invention can be tested by techniques widely known in the state of the art. Examples of techniques for determining the antibacterial activity include, without limitation to, Minimum Bactericidal Concentration (MBC), turbidity decrease bacteriolytic assay, checkerboard/synergy assay, Minimum Inhibitory Concentration (MIC), viable cell counts, time-kill curve, and serum bactericidal titer (SBT). All of these techniques are routine practice for the skilled in the art.

The inventors designed a fusion protein with the Pae87 polypeptide (SEQ ID NO:1) with a C-terminal calcium binding domain (F) for increasing the ability of Pae87 to interact with the Gram-negative outer membrane. The fusion protein Pae87F (SEQ ID NO: 5) displayed an increased bactericidal activity compared with Pae87, which strongly suggested that the C-terminal calcium binding domain interact with the Ca-rich interface of the bacterial surface enhancing antibacterial potential.

So, a second aspect of the present invention related to an isolated polypeptide with antibacterial activity comprising an amino acid sequence with at least 70% identity with the amino acid sequence SEQ ID NO: 2 and a C-terminal calcium binding domain.

The terms "polypeptide", "isolated" and "antibacterial activity" have been previously defined and are fully applicable to this aspect of the invention, as well as all particular and preferred embodiments.

Particularly, the C-terminal calcium binding domain comprising an amino acid sequence SEQ ID NO: 3. So, in a preferred embodiment the polypeptide of the invention comprises an amino acid sequence with at least 70% identity with the amino acid sequence SEQ ID NO: 2 and a C-terminal calcium binding domain wherein the C-terminal calcium binding domain comprises the amino acid sequence SEQ ID NO: 3.

Additionally, the inventors devised five mutations in the sequence of the polypeptide of the invention, obtaining the polypeptide P88 that comprises the amino acid sequence SEQ ID NO: 4, which exerted an improved killing activity on the tested Gram-negative bacteria.

Therefore, in other more preferred embodiment, the polypeptide of the invention comprises the amino acid sequence SEQ ID NO: 4.

Preferably, the polypeptides and derivates with high antimicrobial activity, particularly against Gram-negative bacteria, are the P87, P88 and Pae87F polypeptides. Thus, in another more preferred embodiment, the polypeptide of the invention consists of the amino acid sequence SEQ ID NO: 2, SEQ ID NO: 4 or SEQ ID NO: 5.

In another aspect, the invention relates to a polynucleotide, hereinafter "polynucleotide of the invention", encoding the polypeptide of the invention. Thus, the polynucleotide of the invention is the coding sequence of the polypeptide of the invention. The term "coding sequence" means a polynucleotide, which directly specifies the amino acid sequence of a polypeptide.

The polynucleotide of the invention can be inserted into a nucleic acid construct. The term "nucleic acid construct" means a nucleic acid molecule, either single- or doublestranded, which is isolated from a naturally occurring gene or is modified to contain segments of nucleic acids in a manner that would not otherwise exist in nature or which is synthetic.

Manipulation of the polynucleotide's sequence prior to its insertion into a vector may be desirable or necessary depending on the expression vector. The techniques for modifying polynucleotide sequences utilizing recombinant DNA methods are well known in the art. Thus, in another aspect, the present invention relates to a vector comprising the polynucleotide of the invention, hereinafter the vector of the invention. Preferably, the vector is an expression vector. The term "expression vector" means a linear or circular DNA molecule that comprises a polynucleotide encoding a polypeptide and is operably linked to additional nucleotides (control sequences) that provide for its expression.

The recombinant expression vector may be any vector (e.g., a plasmid or virus) that can be conveniently subjected to recombinant DNA procedures and can bring about expression of the nucleotide sequence. The choice of the vector will typically depend on the compatibility of the vector with the host cell into which the vector is to be introduced. The vectors may be linear or closed circular plasmids.

Besides *Escherichia coli,* for example, useful vectors include, without limiting to, mammal cell-derived expression vectors (e.g., pcDNA3 (Invitrogen) and pEGF-BOS (Mizushima S., Nucleic Acids Res 18(17): 5322 (1990)), pEF, pCDM8), insect cell-derived expression vectors (e.g., "Bac-to-BAC baculovirus expression system" (GIBCO BRL), pBacPAK8), plant-derived expression vectors (e.g., pMH1, pMH2), animal virus-derived expression vectors (e.g., pHSV, pMV, pAdexLcw), retrovirus-derived expression vectors (e.g., pZlpneo), yeast-derived expression vectors (e.g., "Pichia expression kit" (Invitrogen), pNV11, SP-Q01), and *Bacillus subtilis*-derived expression vectors (e.g., pPL608, pKTH50). Examples of vectors to express a polynucleotide in animal cells, such as CHO cells, COS cells or NIH3T3 cells, include, without limiting to, pMAM, pDR2, pBK-RSV, pBK-CMV, pOPRSV, or pOP13. Examples of the virus expression vectors include, without limiting to, attenuated virus hosts such as cowpox or avianpox, Bacille Calmette Guerin (BCG), adenovirus vectors and adeno - associated virus vectors, retrovirus vectors, Salmonella typhi vectors or detoxicated anthrax toxin vectors.

The vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated.

Furthermore, a single vector or plasmid or two or more vectors or plasmids that together contain the total DNA to be introduced into the genome of the host cell, or a transposon, may be used. Thus, other aspect of the present invention relates to a host cell comprising the polypeptide of the invention, the polynucleotide of the invention or the vector of the invention.

The term "host cell" means any cell type that is susceptible to transformation, transfection, transduction, and the like with a nucleic acid construct or expression vector comprising a polynucleotide of the present invention.

The present invention also relates to recombinant host cells, comprising the isolated polynucleotide of the invention, which are advantageously used in the recombinant production of the polypeptides. A vector is introduced into a host cell so that the vector is maintained as a chromosomal integrant or as a self-replicating extra-chromosomal vector as described earlier. The term "host cell" encompasses any progeny of a parent cell that is not identical to the parent cell due to spontaneous mutations.

The host cell may be any cell useful in the recombinant production of a polypeptide of the present invention, e.g., a prokaryote or a eukaryote such as a mammalian, insect, plant, or fungal cell.

In another aspect, the present invention relates to a composition, hereinafter "composition of the invention", comprising the polypeptide, the polynucleotide, the vector or the host cell of the invention. Preferably, the composition of the invention is a pharmaceutical composition and more preferably the composition of the invention comprises a therapeutically effective amount of the polypeptide, polynucleotide, vector or host cell of the invention.

The term "therapeutically effective amount", as used herein, refers to an amount capable of providing a therapeutic effect, and which can be determined by the person skilled in the art by commonly used means. The amount of the polypeptide, polynucleotide, vector or cell of the invention that can be included in the pharmaceutical compositions according to the invention will vary depending on the subject and the particular mode of administration. The therapeutically effective amount will depend on the type of disease caused by a bacterium of the respiratory tract infection, the severity and course of the disease or damage, medical history of the patient and response to the polypeptide, and the discretion of the treating physician. The amount of polypeptide, polynucleotide, vector or cell of the invention is suitably administered to the patient or subject at one time or during a series of treatments. Determining the therapeutically effective amount is routine practice for a person skilled in the art.

According to the conventional techniques known to those skilled in the art, the pharmaceutical composition according to the present invention may be formulated with pharmaceutically acceptable excipient and/or carrier. Thus, in a particular embodiment, the composition of the invention comprises a pharmaceutically acceptable excipient and/or carrier.

The term "excipient" refers to a substance which helps to absorb any of the components of the composition of the invention, stabilizes said components or helps in the preparation of the pharmaceutical composition in the sense of giving it consistency or providing flavours which make them more pleasant. Thus, excipients could have the function of keeping the components bound together, such as for example starches, sugars or celluloses, a sweetening function, a colorant function, the function of protecting the medicament, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material which, included in the galenic forms, is added to the active ingredients or to its associations to enable its preparation and stability, modify its organoleptic properties or determine the physical/chemical properties of the pharmaceutical composition and its bioavailability. The "pharmaceutically acceptable" excipient should allow for the activity of the compounds of the pharmaceutical composition, that is to say, for it to be compatible with said components. Examples of excipients are agglutinants, fillers, disintegrators, lubricants, coaters, sweeteners, flavourings and colorants. Non-limiting, more specific examples of acceptable excipients are starches, sugars, xylitol, sorbitol, calcium phosphate, steroid fats, talc, silica or glycerine, amongst others.

The term "carrier" is preferably an inert substance. The function of the carrier is to facilitate the incorporation of other compounds, to allow a better dosing and administration or to give consistency and form to the pharmaceutical composition. Therefore, the carrier is a substance which is used to dilute any of the components of the composition of the present invention to a determined volume or weight, or even without diluting said components, capable of allowing better dosing and administration or giving consistency and form to the medicament. The carrier is pharmaceutically acceptable. When the form of presentation is liquid, the pharmaceutically acceptable carrier is the diluent.

According to the conventional techniques known to those skilled in the art, the composition provided by this invention may be administered by any appropriate administration route; to this end, said composition will be formulated in the suitable pharmaceutical form for the selected administration route.

As previously explained herein, the inventors have observed that the polypeptide of the invention and the polypeptide Pae87 that comprises the amino acid sequence SEQ ID NO: 1, are able to inhibit the growth or kill bacteria, specially Gram-negative ones, which cause infections and deaths in patients particularly by means of respiratory tract infections. Therefore, the polypeptide of the invention, as well as all the aspects derived from it, and the polypeptide Pae87 comprising the amino acid sequence SEQ ID NO: 1, may be effectively used for the treatment of diseases caused by a bacterium, preferably respiratory tract infections.

Consequently, in another aspect the present invention relates to the polypeptide of the invention or a polypeptide with an amino acid sequence SEQ ID NO: 1, the polynucleotide encoding any of these polypeptides, the vector, the cell, or the composition comprising any of these polypeptides or polynucleotides for use as a medicament.

In another aspect the present invention relates to the polypeptide of the invention or a polypeptide with an amino acid sequence SEQ ID NO: 1, the polynucleotide, the vector, the cell, or the composition of the invention for use in the treatment of a disease caused by a bacterium, preferably an infectious disease, more preferably the disease is a respiratory tract infection.

The terms "polypeptide", "polynucleotide", "vector", "host cell" and "composition" have been previously defined and are fully applicable to these aspects of the invention, as well as all particular and preferred embodiments.

The term "treating", "treat" or "treatment" used interchangeably in the present invention, refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders associated with disease caused by a bacterium, being such disease preferably a respiratory tract infection. The "treatment" of a disorder or disease may also lead to a partial response (e.g., amelioration of symptoms) or complete response (e.g., disappearance of symptoms) of the subject/patient suffering from the disorder or disease.

The term "respiratory tract infection" or "RTI" used herein, refers to a disease involving the respiratory tract infection. The respiratory tract infections are classified as an upper respiratory tract infection (URI or URTI) or a lower respiratory tract infection (LRI or LRTI). The upper respiratory tract is the airway above the glottis or vocal cords, sometimes it is taken as the tract above the cricoid cartilage. This part of the tract includes the nose, sinuses, pharynx, and larynx. The lower respiratory tract consists of the trachea (wind pipe), bronchial tubes, the bronchioles, and the lungs.

Examples of upper respiratory tract infections (URTIs) include, without limitation to, the common cold, laryngitis, pharyngitis/tonsillitis, sinusitis, acute rhinitis, acute rhinosinusitis and acute otitis media. Examples of lower respiratory tract infections (LRTIs) include, without limitation to, acute bronchitis, bronchiolitis, pneumonia and tracheitis.

Causative agents of respiratory tract infections are viral or bacterial, particularly Gram-negative bacteria. Examples of bacteria that cause respiratory tract infections include, without limitation to, *Streptococcus pneumoniae, Mycoplasma pneumoniae, Chlamydia spp, Legionella, Coxiella burnetii, Haemophilus influenzae, Staphylococcus aureus, Streptococcus pyogenes, Pseudomonas aeruginosa, Acinetobacter baumannii, Moraxella catarrhalis, Klebsiella pneumoniae, Escherichia coli, Burkholderia cepacia,* milleri group streptococci and *Mycobacterium tuberculosis.*

The inventors observed that the polypeptide of the invention or polypeptide Pae87 with an amino acid sequence SEQ ID NO: 1, have bactericidal activity, particularly active against Gram-negative bacteria, and more particularly, *P. aeruginosa, A. baumannii,* and *M. catarrhalis.* Therefore, a preferred embodiment of the invention relates to the polypeptide of the invention or a polypeptide with an amino acid sequence SEQ ID NO: 1, the polynucleotide, the vector, the cell, or the composition of the invention for use in the treatment of a disease caused by a bacterium, wherein the bacterium is Gram-negative, and preferably is selected from the list consisting of *Pseudomonas aeruginosa, Acinetobacter baumannii* and *Moraxella catarrhalis.*

As the skilled in the art knows, the antibacterial activity can be non-therapeutically used. Thus, in another aspect the present invention relates to a non-therapeutic use of the polypeptide of the invention or a polypeptide with an amino acid sequence SEQ ID NO: 1, the polynucleotide, the vector, the cell, or the composition of the invention as antibacterial agent.

The terms "polypeptide", "polynucleotide", "vector", "host cell", "composition" and "antibacterial agent" have been previously defined and are fully applicable to the aspect of the invention, as well as all particular and preferred embodiments.

The term "non-therapeutic use" or "not therapeutic use" used herein, refers to use not relating or providing to therapy processes, particularly not relating to diseases caused by a bacterium.

Examples of non-therapeutic use include, without limitation to, food preservation, decontamination, prophylaxis applied to materials, surfaces or devices for different purposes, e.g., medical uses, preventive uses on humans or farm animals, or any other bacterial clearance of certain niches.

In a preferred embodiment of the invention relates to non-therapeutic use of the polypeptide of the invention or a polypeptide with an amino acid sequence SEQ ID NO: 1, the polynucleotide, the vector, the cell, or the composition of the invention as antibacterial agent wherein the bacterium is selected from the list consisting of *Pseudomonas aeruginosa, Acinetobacter baumannii and Moraxella catarrhalis.*

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings are provided by way of illustration and are not intended to be limiting of the present invention.

### DESCRIPTION OF THE DRAWINGS

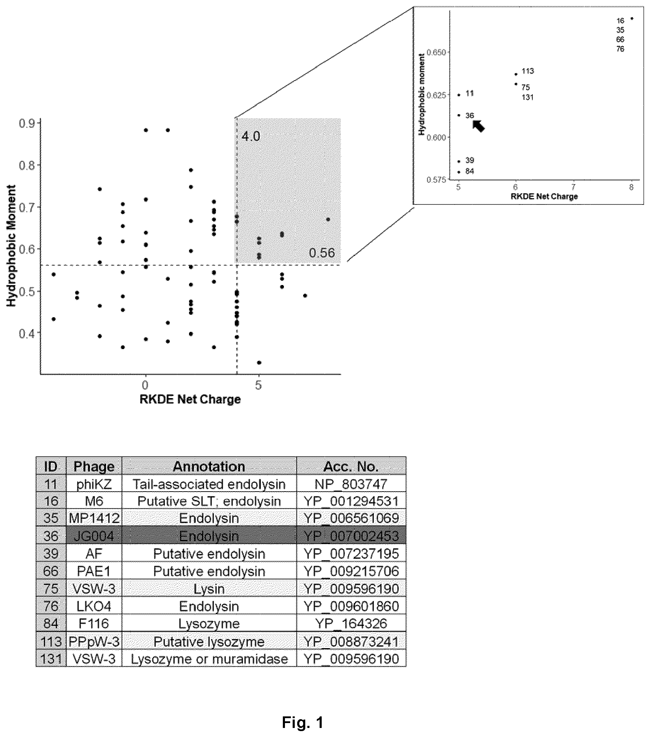

**Figure 1****.** Bioinformatic screening process of suitable anti-Gram-negative phage lysins. Result from the C-terminal physicochemical properties prediction analysis. Each dot represents a single sequence, located within the result space according to its net charge and its hydrophobic moment. The insert shows the final candidates selected according to net charge and hydrophobic moment cutoff values (+4.0 and 0.56, respectively); and table depicting basic information on the final candidates. The endolysin corresponding to Pae87 is highlighted in dark grey.

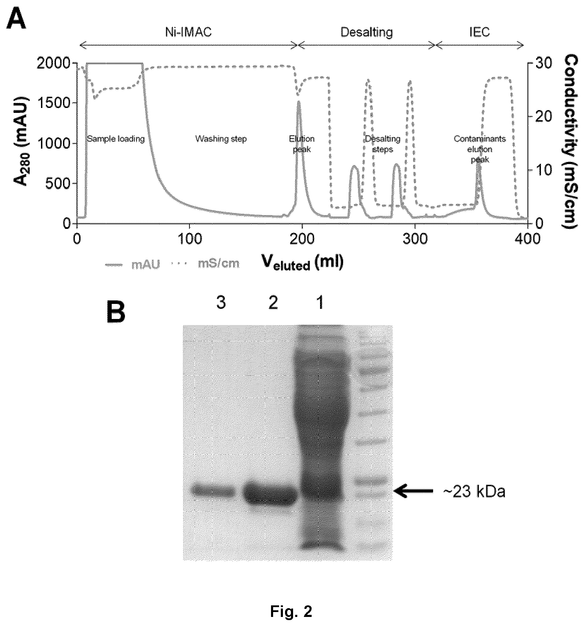

**Figure 2****.** Purification strategy of Pae87. The synthetic gene of JG004 endolysin was synthesized and cloned into a pET28 vector, fusing it to an N-terminal 6xHis tag. A: chromatogram of Pae87 purification process. B: SDS-PAGE of Pae87 purification process. 1: crude expression extract, 2: protein after IMAC, 3: protein after IEC. The purified Pae87 showed an expected molecular mass of around 23 kDa.
**Figure 3****.** Bactericidal activity of Pae87. A: fluorescence microscopy image of the effect of 10 µM Pae87 on a cell suspension of P. aeruginosa PAO1 (up) in comparison with the untreated control (down); B: activity range of Pae87. The bactericidal effect of 10 µM Pae87 in terms of logarithmic viable cell count difference with respect to the control is displayed for a range of clinically relevant bacteria.
**Figure 4****.** Pae87 activity enhancing by fusing a calcium binding domain. A: scheme of the design strategy for an enhanced, calcium-binding variant of Pae87. A C-terminal Excalibur calcium-binding domain from B. subtilis endonuclease YokF was fused at the C-terminal of Pae87 to yield Pae87F synthetic lysin. B: Comparative range of activity of Pae87 and Pae87F.

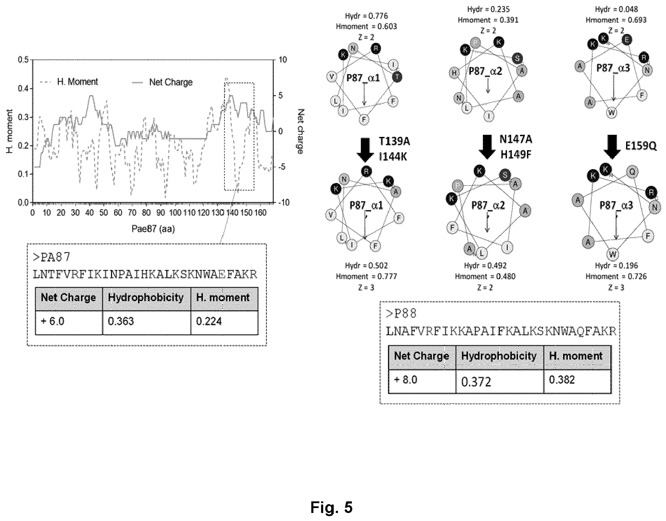

**Figure 5****.** Definition of candidate AMPs P87 and P88 from Pae87 sequence. A: local physicochemical properties profile for Pae87. Hydrophobic moment (left axis) and net charge (right axis) are depicted as calculated in contiguous 11 amino acids windows along Pae87 sequence. The postulated P87 sequence is indicated, as well as the polypeptide sequence and properties. B: possible helices within P87 structure and mutations proposed for net charge and hydrophobic moment maximization in new, synthetic polypeptide P88.
**Figure 6****.** Bactericidal activity of P87 and P88. The bactericidal effect of 10 µM P87/P88 in terms of logarithmic viable cell count difference with respect to the control is displayed for a range of clinically relevant bacteria.

### EXAMPLES

### MATERIALS AND METHODS

### Bacterial strains, plasmids and culture conditions

Bacterial strains used throughout this work are listed in Table 1, whereas plasmids are in Table 2.

**Table 1. Bacterial strains used in this work. ^{a}Hospital Ramon y Cajal (Madrid), donated by Rosa del Campo; ^{b}Centro de Investigaciones Biológicas Margarita Salas; ^{c}Hospital Universitari de Bellvitge (Hospitalet de Llobregat), donated by Carmen Ardanuy; ^{d}Universidad Complutense de Madrid, Facultad de Veterinaria, donated by Juan Miguel Rodríguez.**

| **Species** | **Strain** | **Source** |
|---|---|---|
| *Acinetobacter baumannii* | AB-RYC1 | HRC^{a} |
| | AB-RYC2 | HRC |
| | AB-RYC3 | HRC |
| *Escherichia coli* | BL21(DE3) | CIB^{b} |
| *Moraxella catarrhalis* | MC-RYC1 | HRC |
| | MC-RYC2 | HRC |
| | MC-RYC3 | HRC |
| *Pseudomonas aeruginosa* | PAO1 | CIB |
| | 57.1 | HUB^{c} |
| | 2-006 | HUB |
| | 68.1 | HUB |
| | 126.1 | HUB |
| | 39.5 | HUB |
| | 109.1 | HUB |
| *Streptococcus sp.* (milleri group) | C5-C20 | UCM^{d} |

**Table 2. List of plasmids used.**

| **Name** | **Description** | **Source** |
|---|---|---|
| pUCPA87 | Synthetic construct bearing *pae87* gene between Ndel and HindIII restriction sites. Encodes ampicillin resistance. | GenScript |
| pET28a | Expression vector with N-terminal 6×His tag. Encodes kanamycin resistance. | Invitrogen |
| pETPA87 | Expression vector containing N-terminal 6×His tag fused to *pae87.* Encodes kanamycin resistance. | This work |
| pETPA87-F | Expression vector containing N-terminal 6×His tag fused to *pae87F.* Encodes kanamycin resistance. | GenScript, This work |

All Gram-negative bacteria (*A. baumannii, E. coli, M. catarrhalis, P. aeruginosa*) were cultured in LB medium at 37 °C with 200 rpm shaking, except for *M*. *catarrhalis* which was incubated in the same conditions but in a 5% CO₂ atmosphere. *S*. *milleri* group (SMG) C5-C20 was cultured in BHI medium at 37 °C without shaking.

### Synthetic genes and DNA cloning

All DNA molecular biology techniques were performed essentially as described in J. Sambrook, Molecular Cloning: a Laboratory Manual, Cold Spring Harbor Laboratory Press, 2001. Genes encoding *pae87* and *pae87F* were chemically synthesized by GenScript and provided cloned at Ndel-HindIII restrictions sites in pUCPA87 plasmid or in expression vector pETPA87-F, respectively. The *pae87* gene was subsequently cloned, by Ndel-HindIII restriction sites, into pET28a, yielding pETPA87 plasmid. Plasmids were routinely transformed in *E. coli* DH10B or BL21(DE3) strains for production.

### Dataset construction and analysis

A set of 1252 *Pseudomonas* phage genomes was retrieved from NCBI *Nuccore* database. Such genomes were screened for genes annotated as "lysins, lysozymes, endolysins" and so forth in order to obtain plausible lysin candidate genes (138 in total). Such candidates were subjected to a protein length cutoff to eliminate outliers (only sequences between 50 and 400 amino acids length were kept). Finally, two properties of interest were predicted on the C-terminal 40 amino acids of each sequence, namely net charge and hydrophobic moment. Such properties were calculated using the implementation in R package 'Peptides'.

### Protein expression and purification

All proteins used were produced in *E. coli* BL21(DE3) transformed with the corresponding expression vector. One litre cultures were grown with 50 µg/ml kanamycin until reaching OD600 ≈ 0.6, when cultures were induced with 0.1 mM isopropyl-β-D-thiogalactopyranoside and incubation was resumed overnight at room temperature. Then, cultures were collected and centrifuged (18000 × g, 20 min, 4 °C). Sedimented biomass was resuspended in 20 mM sodium phosphate buffer (NaPiB), pH 7.4, 300 mM NaCl, 40 mM imidazole. Cells were disrupted using a French Press and then centrifuged again in the same conditions to remove cell debris. Proteins were then purified from supernatants by a three-step chromatographic procedure performed in an AKTA start machine: 1) immobilized metal affinity chromatography (IMAC) with nickel as immobilized ligand (HisTrap FF 5 ml columns, GE Healthcare), eluting with 20 mM NaPiB, pH 7.4, 300 mM NaCl, 300 mM imidazole; 2) desalting step (HiTrap Desalting columns 5 ml, GE Healthcare) to change buffer to 20 mM NaPiB, pH 7.4, 150 mM NaCl; 3) ion exchange chromatography (IEC) with DEAE-sepharose columns (HiTrap DEAE FF 5 ml, GE Healthcare). Purified fractions were subjected to SDS-PAGE to check purity and then concentration was calculated by measuring absorbance at 280 nm with the predicted molar extinction coefficients of each protein.

### Synthetic polypeptides

Polypeptides used in this work (*i.e.,* P87 and P88) were purchased from GenScript with a minimum purity of 95%. Polypeptides were diluted in water and their concentration was estimated by measuring UV absorption at 280 nm, taking into account the molar extinction coefficient computed for each polypeptide using ProtParam (ε = 5500 M⁻¹cm⁻¹).

### Bactericidal activity assays

Bactericidal activity of the compounds was assessed by checking viable cell counts after incubation of resting cell suspensions together with each compound with respect to an untreated control. Briefly, OD600 ≈ 0.6 cultures were centrifuged and pellets washed with 20 mM NaPiB pH 6.5, 150 mM NaCl and finally resuspended in the same buffer adjusting to an OD600 ≈ 1.2. One hundred µl of these suspensions were mixed together with 100 µl a buffer solution containing or not 20 µM of the corresponding compound (Pae87, Pae87-F, P87 or P88) for a final concentration of 10 µM, in a 96-well plate. Plates were incubated at 37 °C for 2 h and then samples from each well were serially diluted and plated in LB agar or blood agar to asses viable counts.

Cell suspensions after the 2 h treatment were also sampled for microscopic observation. For this purpose, samples were dyed with *BacLight* LIVE/DEAD kit according to the manufacturer instructions to distinguish between cells with their cell wall compromised and those intact. A Leica DM4000B fluorescence microscope equipped with an L5 (bandpass 480/40) and an N2.1 filter (bandpass 515/60) was used, viewing samples under an HC PL APO 63×/1.40-0.60 oil objective.

### Additional bioinformatic analysis

Further bioinformatic analysis to define potential AMP regions within Pae87 were performed using HeliQuest server to produce local predictions of such properties as net charge, hydrophobicity and hydrophobic moment. A three-dimensional model of Pae87 was produced using homology-guided modelling with SwissProt.

### Statistical methods

All experimental data shown displays a mean ± standard deviation of at least three replicates. Mean comparisons were performed using ANOVA followed by Tukey post-test to asses statistical significance of differences.

### RESULTS

A dataset comprising 138 Pseudomonas phage lysin candidates was examined to obtain the most promising antimicrobial molecules candidates. This was accomplished by predicting net charge and hydrophobic moment along the C-terminal end of such amino acid sequences and then selecting appropriate cutoffs for both properties. The rationale was to maximize positive net charge in order to obtain a molecule able to interact with the negatively charged lipopolysaccharides in the outer surface of Gram-negative bacteria while, at the same time, containing hydrophobic residues that could insert into the outer membrane. In this sense, hydrophobic moment seemed a good variable to optimize, since higher hydrophobic moment values would assure that an increased presence of positively charged, polar residues would be correlated with a geometrically distal accumulation of nonpolar residues. For choosing the cutoff values, net charge and hydrophobic moment distributions were considered as well as properties of previously reported lysins with inherent activity on Gram-negative bacteria from without. A net charge cutoff of ≥ + 4.0 was chosen, which coincides with the third quartile of net charge distribution, and hydrophobic moment cutoff was set in 0.56 (Figure 1). The resulting candidates are shown in Figure 1.

Of such candidates, some of them had been already tested (e.g., ϕKZ phage lysin) and some others were not easily expressed in a soluble form in *E. coli* BL21(DE3) (data not shown). Among those that were well expressed in *E. coli* was phage JG004 phage lysin (YP_007002453), which was subsequently named Pae87. The Pae87 encoding gene was chemically synthesized and purchased from GenScript, and then expressed fused to a 6×His tag from pETPA87 vector. Purification was accomplished by a three-step chromatographic procedure (Figure 2B-C).

Pae87 showed a rather apparent activity against a cellular suspension of P. aeruginosa PAO1 (Figure 3). Such activity included some sort of cell wall permeabilization effect that was observed because of the red fluorescence displayed by Pae87-treated cells, as well as their abnormal morphology (with more spheroid-like shapes) and a certain aggregation (which is a symptom of surface interaction mediated by Pae87). This activity caused a decrease of at least 1 logarithmic unit in viable cells with respect to the control when treating with 10 µM protein. All of the clinical *P. aeruginosa* strains tested were susceptible to Pae87 activity, although with varying killing effects, ranging between 1 and 2.5 logs death (Figure 3B). Other relevant pathogenic bacteria were also susceptible, such as *A. baumannii* or *M*. *catarrhalis,* as well as *Streptococcus* from milleri group.

To try and increase the ability of Pae87 to interact with the Gram-negative outer membrane, a fusion protein was designed bearing a C-terminal calcium binding domain. The Gram-negative outer membrane is stabilized by divalent cations, the Ca-binding domain interact with the Ca-rich interface of the bacterial surface or that, at least, the protein bearing a Ca⁺⁺ have a higher positive net charge and thus its approximation to the negatively charged bacterial surface would be more favoured. A bacterial Ca-binding domain was chosen (*Excalibur*) from a *Bacillus subtilis* endonuclease (Figure 4A-B). The novel fusion protein displayed a similar activity range among our collection of bacteria but an increased bactericidal power compared with Pae87. A 2-fold (and in some cases 3-fold) increase in the logarithmic reduction of viability with respect to the control was observed (Figure 4C), which confirms the superior antibacterial potential of Pae87F.

Finally, the activity of Pae87 towards Gram-negative bacterial surface suggested in Figure 2 was thought to be the result of AMP-like subdomains present at the C-terminal of the protein, motifs that were positively selected in our bioinformatic screening (Figure 1). Indeed, Pae87 seems to contain a C-terminal region of 29 amino acids length in which there is a local maximization of both hydrophobic moment and positive charge (Figure 5A). Such region was termed P87, and a polypeptide with its sequence was synthesized (purchased from GenScript) to test its potential antimicrobial activity. Additionally, P87 possible helices were examined to propose a series of five mutations designed to maximize net charge and hydrophobic moment, yielding polypeptide P88, which was also obtained (Figure 5B).

Both polypeptides were tested on a collection of relevant bacteria (Figure 6). P87 had a bactericidal activity of its own, especially against *P. aeruginosa* (up to 4-log killing) and *A. baumannii* (up to 2-log killing), but P88 activity was much higher. P88 exerted a killing activity ranging between 4 and 6 logarithmic units of viable cells reduction in all of the three tested Gram-negative susceptible bacteria (*P. aeruginosa, M. catarrhalis* and *A. baumannii*). Therefore, the mutations introduced in P88 increased its bactericidal activity, suggesting that the maximization of such physicochemical properties is a valid way to improve the interaction with the bacterial surface.

### CONCLUSIONS

An effective bioinformatic procedure for data mining of therapeutically relevant lysins was devised. This way, a lysin from a *Pseudomonas aeruginosa* phage, Pae87, has been proposed as an antimicrobial, active both against *P. aeruginosa* and *A. baumannii,* as well as other relevant pathogens such as *M. catarrhalis* or milleri group streptococci. A novel enhancement strategy of lysins activity was proposed, fusing Pae87 to a calcium-binding domain. The resulting molecule, Pae87F, displayed an improved activity against the same range of bacteria. Finally, an AMP-like region was defined from Pae87 and polypeptides derived from it were synthesized and tested for antimicrobial abilities. The wild type P87 polypeptide was proved to have an antimicrobial activity of its own. A procedure based on positive net charge and hydrophobic moment optimization was used to improve P87 activity, yielding the much more active polypeptide P88.

## Claims

1. An isolated polypeptide with antibacterial activity comprising an amino acid sequence with at least 70% identity with the amino acid sequence SEQ ID NO: 2 wherein the amino acid sequence is not SEQ ID NO: 1.

2. An isolated polypeptide with antibacterial activity comprising an amino acid sequence with at least 70% identity with the amino acid sequence SEQ ID NO: 2 and a C-terminal calcium binding domain.

3. The polypeptide according to claim 2, wherein the C-terminal calcium binding domain comprising the amino acid sequence SEQ ID NO: 3.

4. The polypeptide according to any one of claims 1 to 3, wherein the polypeptide comprising the amino acid sequence SEQ ID NO: 4.

5. The polypeptide according to any one of claims 1 to 4, wherein the polypeptide consisting of the amino acid sequence SEQ ID NO: 2, SEQ ID NO: 4 or SEQ ID NO: 5.

6. A polynucleotide encoding the polypeptide according to any one of claims 1 to 5.

7. A vector comprising the polynucleotide according to claim 6.

8. A host cell comprising the polypeptide according to any one of claims 1 to 5, the polynucleotide according to claim 6 or the vector according to claim 7.

9. A composition comprising the polypeptide according to any one of claims 1 to 5, the polynucleotide according to claim 6, the vector according to claim 7 or the host cell according to claim 8.

10. The composition according to claim 9 wherein the composition is a pharmaceutical composition, preferably the composition further comprising a pharmaceutically acceptable excipient and/or carrier.

11. A polypeptide according to any one of claims 1 to 5 or a polypeptide with an amino acid sequence SEQ ID NO: 1, the polynucleotide according to claim 6, the vector according to claim 7, the host cell according to claim 8 or the composition according to claim 9 or 10 for use as a medicament.

12. A polypeptide according to any one of claims 1 to 5 or a polypeptide with an amino acid sequence SEQ ID NO: 1, the polynucleotide according to claim 6, the vector according to claim 7, the host cell according to claim 8 or the composition according to claim 9 or 10 for use in the treatment of a disease caused by a bacterium, preferably the disease is a respiratory tract infection.

13. The polypeptide for use according to claim 12, wherein the bacterium is selected from the list consisting of *Pseudomonas aeruginosa, Acinetobacter baumannii* and *Moraxella catarrhalis.*

14. Non therapeutic use of the polypeptide according to any one of claims 1 to 5 or a polypeptide with an amino acid sequence SEQ ID NO: 1, the polynucleotide according to claim 6, the vector according to claim 7, the host cell according to claim 8 or the composition according to claim 9 or 10 as antibacterial agent.

15. Use according to claim 14 wherein the bacterium is selected from the list consisting of *Pseudomonas aeruginosa, Acinetobacter baumannii* and *Moraxella catarrhalis.*
